# EUROPEAN PATENT SPECIFICATION

(11) **EP 3 544 545 B1**
(45) Date of publication and mention of the grant of the patent: **23.12.2020**
(21) Application number: 17817457.9
(22) Date of filing: 22.11.2017
(51) Int. Cl.: A61F 2/01

(54) **EMBOLIC PROTECTION DEVICE**
EMBOLIESCHUTZVORRICHTUNG
DISPOSITIF DE PROTECTION EMBOLIQUE

(30) Priority: 22.11.2016 US 201662425276 P
(43) Date of publication of application: 02.10.2019
(73) Proprietor: Boston Scientific Scimed, Inc., Maple Grove, MN 55311 (US)
(72) Inventor: EGGERT, Joel T., Plymouth, Minnesota 55442 (US); PAGORIA, Douglas D., Forest Lake, Minnesota 55025 (US); WULFMAN, David R., Minneapolis, Minnesota 55405 (US); PENNINGTON, Douglas, Stillwater, Minnesota 55082 (US); ROHL, James P., Prescott, Minnesota 54021 (US); SCHACHTNER, Joseph J., Saint Paul, Minnesota 55106 (US)
(74) Representative: Vossius & Partner Patentanwälte Rechtsanwälte mbB
(86) International application number: PCT/US2017/063065
(87) International publication number: WO 2018/098317

(56) References cited:
- WO-A1-2008/066881
- WO-A2-02/47539
- US-A1- 2010 168 785

## Description

### Technical Field

The present disclosure pertains to medical devices, and methods for manufacturing and/or using medical devices. More particularly, the present disclosure pertains to an embolic protection device and/or apparatus and methods of manufacture therefor.

### Background

A wide variety of intracorporeal medical devices have been developed for medical use, for example, surgical and/or intravascular use. Some of these devices include guidewires, catheters, medical device delivery systems (e.g., for stents, grafts, replacement valves, etc.), and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and/or using medical devices.

WO 2008/066881 A1 discloses an embolic protection device comprising a filter that comprises a porous mesh material defining a collection chamber for captured emboli. The filter has a deployed condition wherein an outer periphery of the filter contacts a blood vessel wall to direct blood flow and potential emboli into the collection chamber. The embolic protection device further comprises a catheter port through the filter configured for passage of a catheter shaft through the catheter port, and a resilient seal positioned within the catheter port and configured for forming a seal around a catheter shaft placed through the catheter port.

### Summary

According to the invention, an aortic arch embolic protection device as defined by claim 1 is provided. The dependent claims define embodiments.

In a first aspect, an aortic arch embolic protection device having an upstream end and a downstream end may comprise an anchoring structure comprising a first anchoring ring at the upstream end and a second anchoring ring at the downstream end. A first filter element may extend from the first anchoring ring to the second anchoring ring and defining a lumen through the aortic arch embolic protection device. A second filter element may be disposed within the lumen of the first filter element. The second filter element may include an access port configured to facilitate passage of an interventional catheter through the access port.

In addition or alternatively, and in a second aspect, the access port is disposed proximate an upstream end of the second filter element.

In addition or alternatively, and in a third aspect, the first anchoring ring includes a first self-expanding stent structure.

In addition or alternatively, and in a fourth aspect, the second anchoring ring includes a second self-expanding stent structure.

In addition or alternatively, and in a fifth aspect, openings formed through the first filter element are smaller than openings formed through the second filter element.

In addition or alternatively, and in a sixth aspect, an upstream end of the second filter element is connected to the first anchoring ring.

In addition or alternatively, and in a seventh aspect, an upstream end of the second filter element includes a plurality of tethers connecting the second filter element to the first anchoring ring.

In addition or alternatively, and in an eighth aspect, the access port is biased toward a closed configuration.

In addition or alternatively, and in a ninth aspect, the access port is configured to compress against an exterior surface of the interventional catheter when the interventional catheter is translated through the access port.

In addition or alternatively, and in a tenth aspect, fluid pressure against a radially outward surface of the second filter element as a result of fluid flow through the lumen urges the access port toward a closed configuration.

In addition or alternatively, and in an eleventh aspect, the access port includes an elastic band.

In addition or alternatively, and in a twelfth aspect, an aortic arch embolic protection device having an upstream end and a downstream end may comprise an anchoring structure comprising a first anchoring ring at the upstream end, a second anchoring ring at the downstream end, and a tubular scaffold extending from the first anchoring ring to the second anchoring ring. A first filter element may extend along the tubular scaffold from the first anchoring ring to the second anchoring ring and defining a lumen through the aortic arch embolic protection device. A second filter element may extend from the second anchoring ring upstream to an access port configured to facilitate passage of an interventional catheter through the access port. At least a portion of the second filter element may be disposed within the lumen of the first filter element and radially spaced from the first filter element.

In addition or alternatively, and in a thirteenth aspect, the first filter element includes a porous polymeric membrane.

In addition or alternatively, and in a fourteenth aspect, the second filter element includes a porous polymeric membrane.

In addition or alternatively, and in a fifteenth aspect, the first filter element is radially spaced from the tubular scaffold.

In addition or alternatively, and in a sixteenth aspect, an aortic arch embolic protection device having an upstream end and a downstream end may comprise an anchoring structure comprising a first anchoring ring at the upstream end and a second anchoring ring at the downstream end. A first filter element may extend from the first anchoring ring to the second anchoring ring and defining a lumen through the aortic arch embolic protection device. A second filter element may extend transversely across the lumen of the first filter element at a plurality of axial locations along a longitudinal axis of the lumen. The second filter element may include an access port configured to facilitate passage of an interventional catheter through the access port.

In addition or alternatively, and in a seventeenth aspect, the second filter element comprises a porous media extending along a majority of a length of the lumen.

In addition or alternatively, and in an eighteenth aspect, the porous media defines an effective porosity over its entire length that is less than a porosity of the porous media in any given cross-section of the porous media.

In addition or alternatively, and in a nineteenth aspect, the second filter element comprises a series of porous media sections distributed along a length of the lumen.

In addition or alternatively, and in a twentieth aspect, each porous media section of the series of porous media sections is spaced apart from an adjacent porous media section.

The above summary of some embodiments, aspects, and/or examples is not intended to describe each embodiment or every implementation of the present disclosure. The figures and the detailed description which follows more particularly exemplify these embodiments.

### Brief Description of the Drawings

The disclosure may be more completely understood in consideration of the following detailed description of various embodiments in connection with the accompanying drawings, in which:
FIG. 1 is a partial view of elements of an example heart and aorta;
FIG. 2 illustrates an example aortic arch embolic protection device;
FIG. 3 illustrates the example aortic arch embolic protection device of FIG. 2 with an interventional catheter extending therethrough;
FIG. 4 illustrates an example aortic arch embolic protection device;
FIG. 5 illustrates an example aortic arch embolic protection device;
FIG. 6 illustrates the example aortic arch embolic protection device of FIG. 5 with an interventional catheter extending therethrough;
FIG. 7 illustrates an example aortic arch embolic protection device;
FIGS. 8 and 8A are cross-sectional views of the example aortic arch embolic protection device of FIG. 7;
FIG. 9 illustrates the example aortic arch embolic protection device of FIG. 7 with an interventional catheter extending therethrough; and
FIG. 10 illustrates an example aortic arch embolic protection device.

### Detailed Description

The following description should be read with reference to the drawings, which are not necessarily to scale, wherein like reference numerals indicate like elements throughout the several views. The detailed description and drawings are intended to illustrate but not limit the claimed invention. Those skilled in the art will recognize that the various elements described and/or shown may be arranged in various combinations and configurations without departing from the scope of the disclosure. The detailed description and drawings illustrate example embodiments of the claimed invention.

For the following defined terms, these definitions shall be applied, unless a different definition is given in the claims or elsewhere in this specification.

All numeric values are herein assumed to be modified by the term "about," whether or not explicitly indicated. The term "about", in the context of numeric values, generally refers to a range of numbers that one of skill in the art would consider equivalent to the recited value (e.g., having the same function or result). In many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g., 1 to 5 includes 1, 1.5, 2, 2.75, 3, 3.80, 4, and 5).

Although some suitable dimensions, ranges, and/or values pertaining to various components, features and/or specifications are disclosed, one of skill in the art, incited by the present disclosure, would understand desired dimensions, ranges, and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For simplicity and clarity purposes, not all elements of the disclosed invention are necessarily shown in each figure or discussed in detail below. However, it will be understood that the following discussion may apply equally to any and/or all of the components for which there are more than one, unless explicitly stated to the contrary. Additionally, not all instances of some elements or features may be shown in each figure for clarity.

Relative terms such as "proximal", "distal", "advance", "retract", variants thereof, and the like, may be generally considered with respect to the positioning, direction, and/or operation of various elements relative to a user/operator/manipulator of the device, wherein "proximal" and "retract" indicate or refer to closer to or toward the user and "distal" and "advance" indicate or refer to farther from or away from the user. In some instances, the terms "proximal" and "distal" may be arbitrarily assigned in an effort to facilitate understanding of the disclosure, and such instances will be readily apparent to the skilled artisan. Other relative terms, such as "upstream", "downstream", "inflow", and "outflow" refer to a direction of fluid flow within a lumen, such as a body lumen, a blood vessel, or within a device.

The term "extent" may be understood to mean a greatest measurement of a stated or identified dimension. For example, "outer extent" may be understood to mean a maximum outer dimension, "radial extent" may be understood to mean a maximum radial dimension, "longitudinal extent" may be understood to mean a maximum longitudinal dimension, etc. Each instance of an "extent" may be different (e.g., axial, longitudinal, lateral, radial, circumferential, etc.) and will be apparent to the skilled person from the context of the individual usage. Generally, an "extent" may be considered a greatest possible dimension measured according to the intended usage. In some instances, an "extent" may generally be measured orthogonally within a plane and/or cross-section, but may be, as will be apparent from the particular context, measured differently - such as, but not limited to, angularly, radially, circumferentially (e.g., along an arc), etc.

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to effect the particular feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangeable with each other to form other additional embodiments or to complement and/or enrich the described embodiment(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously-used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

Preventing emboli and other debris from entering the carotid arteries (such as the brachiocephalic artery, or right common carotid artery, the left common carotid, and the left subclavian) by way of the aorta may reduce the incidence of ischemic stroke. Emboli and other debris in the aorta may come from several sources. These sources may include: 1) aortic atheroma which detaches from the wall of the aorta due to various reasons including incising, clamping, and/or clamp release of the aorta during surgery; 2) debris released during surgery on the heart such as the installation of a replacement heart valve or to access the left atrial appendage; 3) thrombus which forms in the left atrium or the left atrial appendage resulting from atrial fibrillation; 4) thrombus which forms on ventricular assist devices; 5) venous thrombus which passes into the left ventricle through a patent foramen ovale or other arteriovenous shunt; and 6) other less common sources.

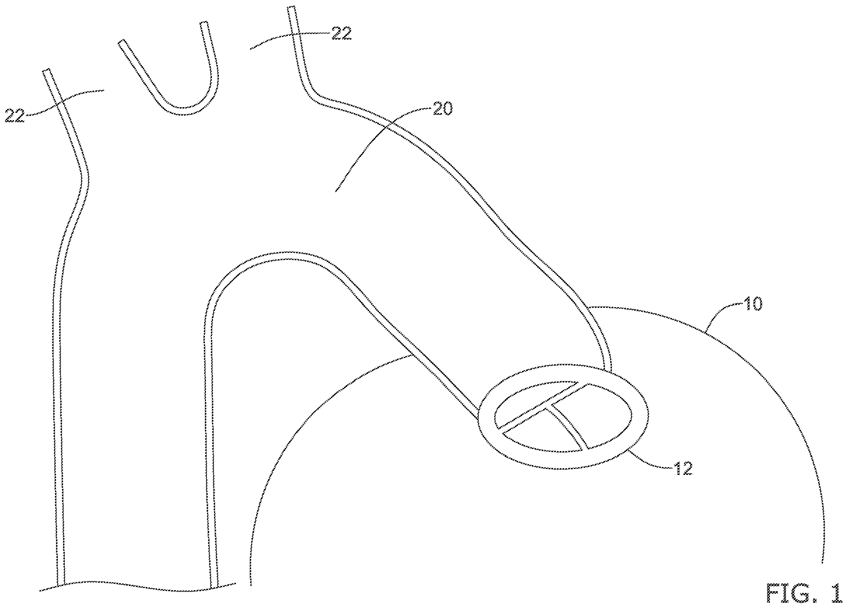

FIG. 1 illustrates a posterior view of an aorta 20 extending from an aortic valve 12 of an example heart 10. Arteries 22 (e.g., subclavian arteries, carotid arteries, brachiocephalic artery) extend from the arch of the aorta 20 to important internal organs. Disclosed herein are medical devices and/or methods that may be used within a portion of the cardiovascular system (e.g., the arch of the aorta 20, etc.) in order to capture thrombi and/or embolic material, and/or prevent the passage of thrombi and/or embolic material downstream, for example, into the arteries 22 while providing passage for a medical device used to treat certain conditions (e.g., a replacement heart valve system, a left atrial appendage occlusion device, etc.). The devices disclosed herein may also provide a number of additional desirable features and benefits as described in more detail below. For the purpose of this disclosure, the discussion below is directed toward use in the arch of the aorta 20 and will be so described in the interest of brevity. This, however, is not intended to be limiting as the skilled person will recognize that the following discussion may also apply to other vessels and/or treatment locations within a patient with no or minimal changes to the structure and/or scope of the disclosure.

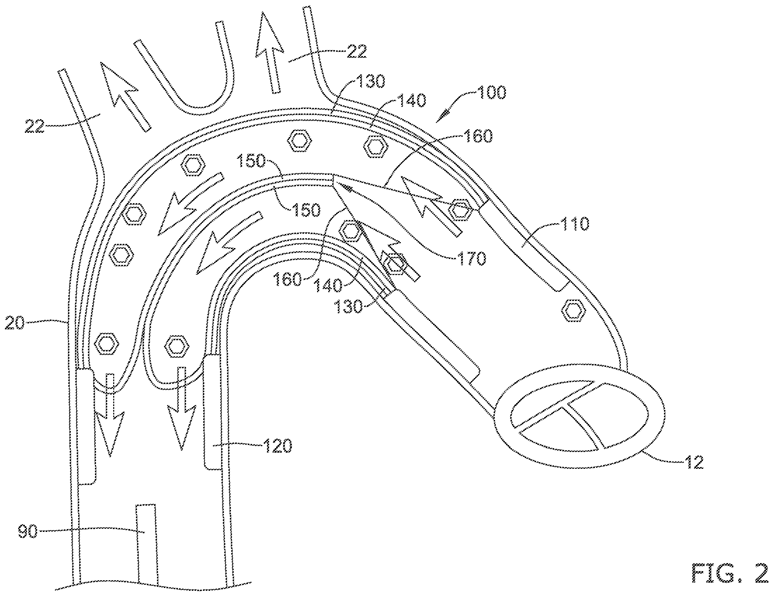

FIGS. 2-3 illustrates an example aortic arch embolic protection device 100 disposed within the arch of the aorta 20 proximate the aortic valve 12. As shown in FIGS. 2-3, the aortic arch embolic protection device 100 may be disposed and/or extend across the entrance/annulus of the arteries 22. The aortic arch embolic protection device 100 may have an upstream end and a downstream end generally corresponding to a direction of fluid/blood flow within the aorta 20 or other treatment location, as indicated by the arrows illustrated.

The aortic arch embolic protection device 100 may include an anchoring structure comprising a first anchoring ring 110 at the upstream end, a second anchoring ring 120 at the downstream end, and a tubular scaffold 130 extending from the first anchoring ring 110 to the second anchoring ring 120. The tubular scaffold 130 may include an expandable stent-like structure, a metallic grid, a polymeric grid, a woven structure, etc. In some embodiments, the tubular scaffold 130 may include longitudinally and/or circumferentially oriented struts and/or filaments. Generally speaking, openings through and/or cells formed within the tubular scaffold 130 may be large relative to other structures, elements, and/or devices described herein to facilitate fluid flow therethrough. In some embodiments, the first anchoring ring 110 may include a first self-expanding stent structure, the second anchoring ring 120 may include a second self-expanding stent strucutre, and/or the tubular scaffold 130 may include a self-expanding stent structure. Other configurations, including but not limited to a balloon expandable structure and/or a mechanically expandable structure, are also contemplated. In some embodiments, the first anchoring ring 110 and/or the second anchoring ring 120 may be formed as an annular ring configured to exert a radially outward force upon a wall of the arch of the aorta 20 to anchor the aortic arch embolic protection device 100 and/or to prevent migration thereof. In some embodiments, the first anchoring ring 110 and/or the second anchoring ring 120 may be fixedly attached to the tubular scaffold 130. In at least some embodiments, the first anchoring ring 110 and/or the second anchoring ring 120 may be integrally formed with the tubular scaffold 130 as a single unitary stent structure. In some embodiments, the first anchoring ring 110, the second anchoring ring 120, and the tubular scaffold 130 may collectively form the anchoring structure and/or a single anchoring structure. In other words, the tubular scaffold 130 may also form and/or be an anchoring element. Some suitable but non-limiting materials for the anchoring structure, the first anchoring ring 110, the second anchoring ring 120, and/or the tubular scaffold 130 are described below.

The aortic arch embolic protection device 100 may include a first filter element 140 extending along the tubular scaffold 130 from the first anchoring ring 110 to the second anchoring ring 120. The first filter element 140 may define a lumen extending axially through the aortic arch embolic protection device 100. In some embodiments, the first filter element 140 may include a porous polymeric membrane and/or a fine braided mesh having a plurality of openings or pores extending and/or formed therethrough. The plurality of openings or pores extending and/or formed through the porous polymeric membrane and/or the fine braided mesh of the first filter element 140 may be smaller than the openings and/or cells of the tubular scaffold 130 to facilitate the capture of thrombi and/or embolic material, and/or to prevent thrombi and/or embolic material from traveling into the arteries 22. While illustrated in a partial cut-away view, the skilled practitioner will recognize that the first filter element 140 may extend circumferentially about a longitudinal axis of the anchoring structure and/or the tubular scaffold 130. In at least some embodiments, the first filter element 140 may extend around an entire inner circumference of the anchoring structure and/or the tubular scaffold 130. Some suitable but non-limiting materials for the first filter element 140 are described herein.

The aortic arch embolic protection device 100 may include a second filter element 150, wherein at least a portion of the second filter element 150 is disposed within the lumen of the first filter element 140. In some embodiments, the second filter element 150 may be fixedly and/or directly attached to the second anchoring ring 120. In some embodiments, the second filter element 150 may extend upstream of the second anchoring ring 120 toward the first anchoring ring 110. In some embodiments, at least a portion of the second filter element 150 may be radially spaced from the first filter element 140 and/or the tubular scaffold 130. In some embodiments, the second filter element 150 may include a porous polymeric membrane and/or a fine braided mesh having a plurality of openings or pores extending therethrough. The plurality of openings or pores extending and/or formed through the porous polymeric membrane and/or the fine braided mesh of the second filter element 150 may be larger than the openings or pores extending and/or formed through the first filter element 140 and/or the cells of the tubular scaffold 130 to facilitate the capture of thrombi and/or embolic material, and/or to prevent thrombi and/or embolic material from traveling downstream in the aorta 20, while maintaining proper and/or necessary fluid/blood flow characteristics (e.g., volume, pressure, etc.) therein. In other words, the plurality of openings or pores extending and/or formed through the first filter element 140 may be smaller than the plurality of openings or pores extending and/or formed through the second filter element 150. Some suitable but non-limiting materials for the second filter element 150 are described herein.

In some embodiments, the second filter element 150 may form a sack or bag-like receptacle at a downstream portion of the second filter element 150. The downstream portion of the second filter element 150 may extend downstream from the second anchoring ring 120 before and/or while extending radially inward toward a longitudinal axis of the lumen of the first filter element 140. In other words, at least a portion of the second filter element 150 may extend and/or be disposed downstream of a point of attachment of the second filter element 150 to the second anchoring ring 120, particularly when the aortic arch embolic protection device 100 is disposed within the arch of the aorta 20 and/or at another treatment location with fluid/blood flowing therethrough. The downstream portion of the second filter element 150 may turn and/or extend upstream along the longitudinal axis of the lumen of the first filter element 140, thereby forming a tubular structure extending upstream along the longitudinal axis of the lumen of the first filter element 140 toward the first anchoring ring 110.

In some embodiments, the second filter element 150 may include an access port 170 configured to facilitate passage of an interventional catheter 90 through the access port 170. In some embodiments, the second filter element 150 may extend from the second anchoring ring 120 upstream to the access port 170. In other words, the access port 170 may be located and/or disposed upstream of the second anchoring ring 120 proximate an upstream end of the second filter element 150. Generally speaking, the access port 170 may be disposed downstream of the first anchoring ring 110. In some embodiments, the upstream end of the second filter element 150 may be connected to the first anchoring ring 110. For example, the upstream end of the second filter element 150 may include a plurality of tethers 160 connecting the second filter element 150 to the first anchoring ring 110. Some suitable but non-limiting materials for the plurality of tethers 160 are described below.

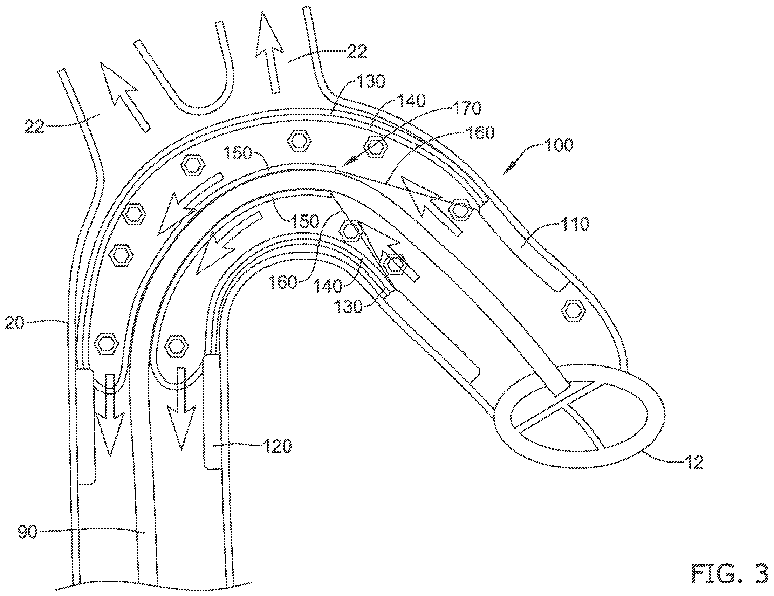

In some embodiments, the access port 170 may be biased toward a closed configuration. In some embodiments, fluid pressure against a radially outward surface of the second filter element 150 as a result of fluid/blood flow through the lumen of the first filter element 140 may urge and/or bias the access port 170 toward the closed configuration, as seen in FIG. 2 for example. In some embodiments, advancement and/or translation of the interventional catheter 90 upstream through the aortic arch embolic protection device 100 and/or the access port 170 may urge and/or actuate the access port 170 into an open configuration. The access port 170 may be configured to compress against an exterior surface of the interventional catheter 90 when the interventional catheter 90 is advanced and/or translated through the access port 170. In some embodiments, when the aortic arch embolic protection device 100 is disposed within the arch of the aorta 20 or at another treatment location, with fluid/blood flowing therethrough, the access port 170 may only attain the open configuration when the interventional catheter 90 or another similar device is disposed within and/or through the access port 170, thereby urging and/or forcing the access port 170 into the open configuration, as seen in FIG. 3 for example.

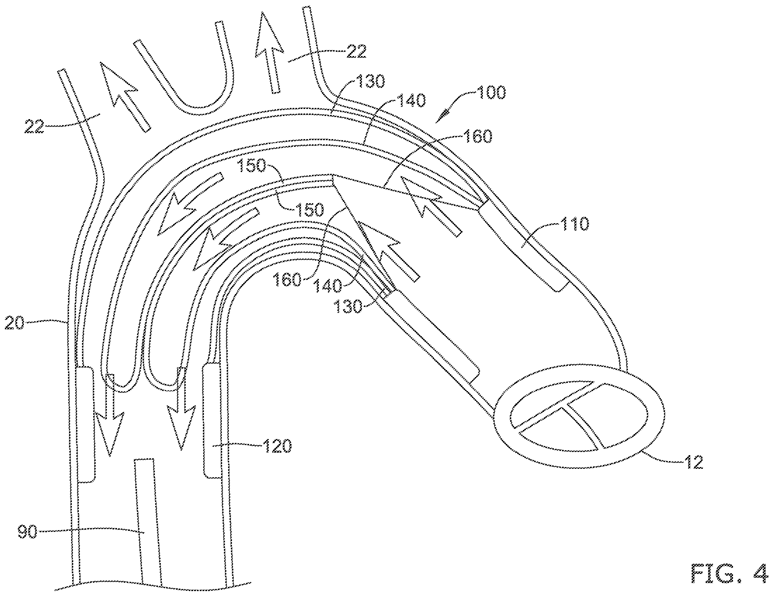

As shown in FIGS. 2 and 3, for example, the first filter element 140 and the second filter element 150 may be separate and independent structures from one another. In other words, the first filter element 140 and the second filter element 150 may lack direct contact with each other and/or may not be directly connected together. However, in some embodiments, the first filter element 140 and the second filter element 150 may be directly connected together at and/or adjacent a downstream end of the aortic arch embolic protection device 100, and/or the first filter element 140 and the second filter element 150 may be integrally formed as a single structure, as shown in FIG. 4. In some embodiments, the first filter element 140 may be radially spaced and/or held away from the tubular scaffold 130. For example, additional downstream tethers may be used to substantially center the first filter element 140 and the second filter element 150 along the longitudinal axis of the aortic arch embolic protection device 100. In some embodiments, neither the first filter element 140 nor the second filter element 150 is directly attached to the second anchoring ring 120. By spacing the first filter element 140 away from the tubular scaffold 130 and/or the wall of the arch of the aorta 20, as shown in FIG. 4, an increased surface area may be obtained for filtering thrombi and/or embolic material while limiting the effect of the filter elements/membrane on fluid/blood flow through the aortic arch embolic protection device 100.

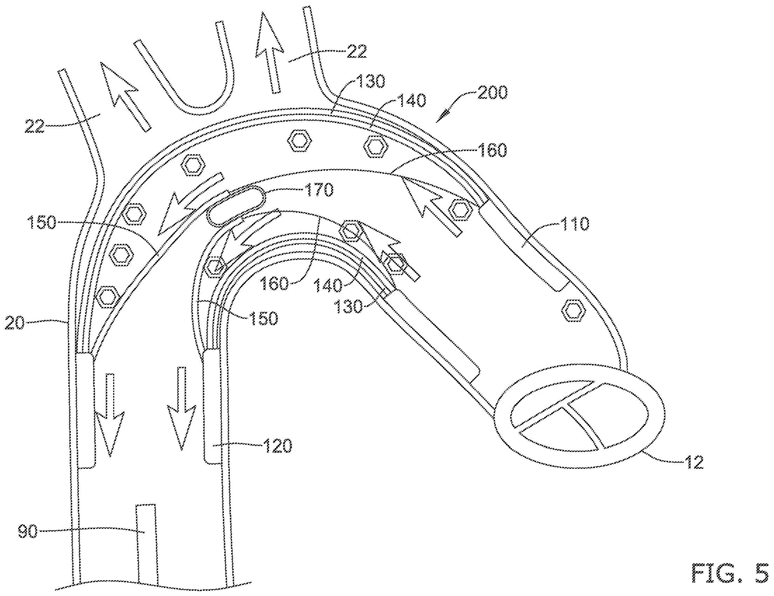

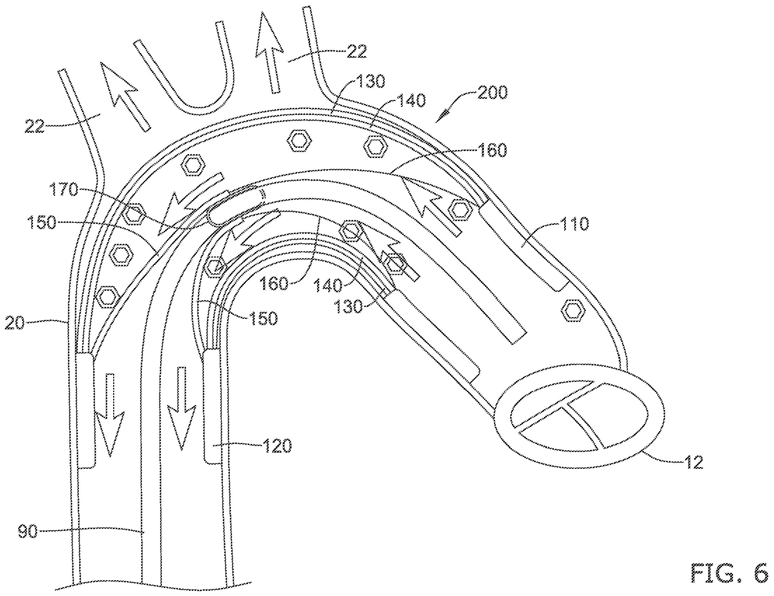

A similar arrangement to that shown in FIGS. 2 and 3 may be seen, for example, in the aortic arch embolic protection device 200 FIGS. 5 and 6. Generally speaking, the description above also applies to the configuration of FIGS. 5 and 6. In addition or alternatively, the access port 170 may include an elastic band. The access port 170 and/or the elastic band may be resiliently biased toward the closed configuration. In some embodiments, the access port 170 and/or the elastic band may close and/or compress radially inwardly upon itself in the closed configuration. In some embodiments, the access port 170 and/or the elastic band may include a hemostatic valve and/or other suitable fluid-blocking element disposed within the access port 170 and/or the elastic band to prevent fluid/blood and any thrombi or embolic material therein from passing through the access port 170 and/or the elastic band. Instead, fluid/blood is directed through the second filter element 150, where any thrombi and/or embolic material is captured and/or removed from the fluid/blood, which is permitted to pass through the second filter element 150 and continue flowing downstream in the aorta 20. In at least some embodiments, the access port 170 and/or the elastic band may include a plurality of tethers 160 connecting the second filter element 150, the access port 170, and/or the elastic band to the first anchoring ring 110.

As above, advancement and/or translation of the interventional catheter 90 upstream through the aortic arch embolic protection device 200 and/or the access port 170 may urge and/or actuate the access port 170 and/or the elastic band into an open configuration. The access port 170 and/or the elastic band may be configured to compress against an exterior surface of the interventional catheter 90 when the interventional catheter 90 is advanced and/or translated through the access port 170 and/or the elastic band. In some embodiments, when the aortic arch embolic protection device 200 is disposed within the arch of the aorta 20 or at another treatment location, with fluid/blood flowing therethrough, the access port 170 and/or the elastic band may only attain the open configuration when the interventional catheter 90 or another similar device is disposed within and/or through the access port 170 and/or the elastic band, thereby urging and/or forcing the access port 170 and/or the elastic band into the open configuration, as seen in FIG. 6 for example. Some suitable but non-limiting materials for the elastic band are described herein.

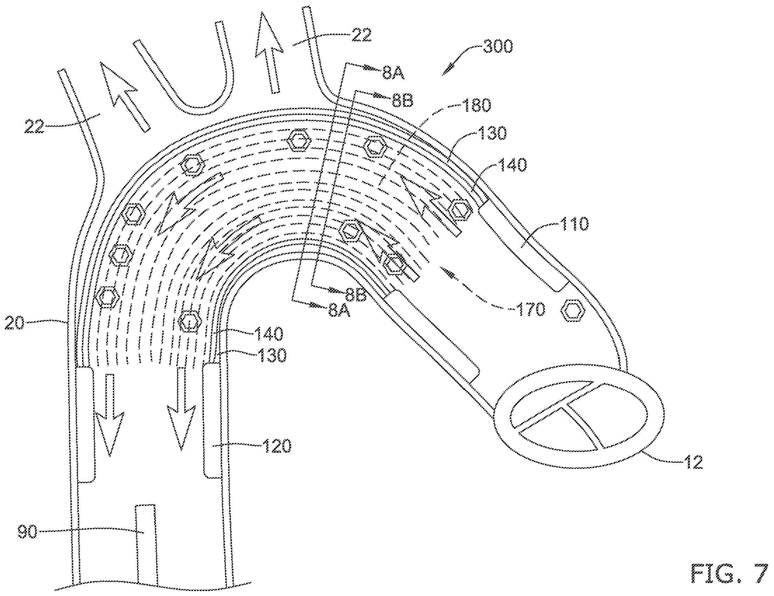

FIGS. 7-9 illustrate an example aortic arch embolic protection device 300 having substantially the same anchoring structure (e.g., first anchoring ring 110, second anchoring ring 120, and/or tubular scaffold 130, etc.) and first filter element 140 as that described above. In addition or alternatively, in some embodiments, the aortic arch embolic protection device 300 may include a second filter element 180 extending transversely across the lumen of the first filter element 140 at a plurality of axial locations along a longitudinal axis of the lumen of the first filter element 140. In other words, a plurality of transverse sections taken at different axial locations along the length of the lumen of the first filter element 140 perpendicular to the longitudinal axis of the lumen of the first filter element 140 may each have the second filter element 180 extending across the inner diameter of the lumen of the first filter element 140. In some embodiments, the second filter element 180 may comprise a porous media extending along a majority of a length of the lumen of the first filter element 140. The porous media may take one or more of a number of different forms. For example, the porous media may include a plurality of layers of porous membranes, a plurality of layers of woven material, a fine braided mesh, a plurality of layers of intersecting filaments and/or filamentous material, a sponge-like material, or other suitable arrangements. In at least some embodiments, the porous media may be generally rigid and/or self-supporting. In some embodiments, the second filter element 180 may be fixedly attached to the first filter element 140. In some embodiments, the second filter element 180 may be bonded and/or laminated to the first filter element 140. Other suitable attachment means are also contemplated. Some suitable but non-limiting materials for the second filter element 180 and/or the porous media are described below.

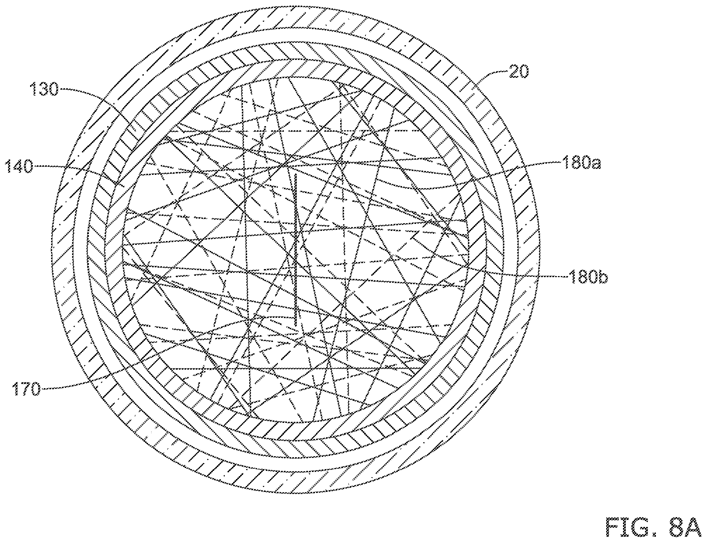

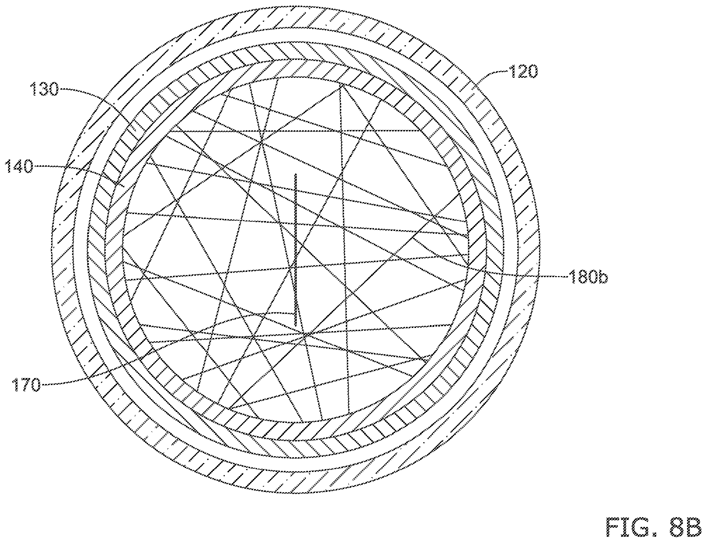

The porous media may define an effective porosity over its entire length that is less than a porosity of the porous media in any given cross-section of the porous media. Stated differently, different cross-sections of the porous media may occlude and/or filter different portions of the lumen of the first filter element 140, as illustrated by the cross-sections shown in FIGS. 8A and 8B. FIG. 8A shows two example cross-sections of the second filter element 180a and 180b (collectively second filter element 180) each having a plurality of intersecting filaments and/or filamentous material. For clarity, the cross-section of the second filter element 180b shown in FIG. 8B is shown in phantom. FIG. 8B shows only one of the two example cross-sections of the second filter element 180b. As may be discerned from FIG. 8A, each individual filament extends transversely across the lumen of the first filter element 140 at a different location, thereby reducing the effective porosity of the overall structure. For example, the openings seen in the cross-section of FIG. 8A are smaller than the openings seen in the cross-section of FIG. 8B because the filaments disposed in the cross-section at line 8A-8A of FIG. 7 intersect the openings between the filaments disposed in the cross-section at line 8B-8B of FIG. 7.

Functionally, thrombi and/or embolic material having a cross-sectional size greater than the effective porosity of porous media will be unable to pass through the second filter element 180 without being captured because no straight, unobstructed path through the entire second filter element 180 and/or the entire porous media exists that is larger than the effective porosity. As thrombi and/or embolic material enter into the second filter element 180, it becomes entangled in the porous media and fluid/blood flows through the porous media.

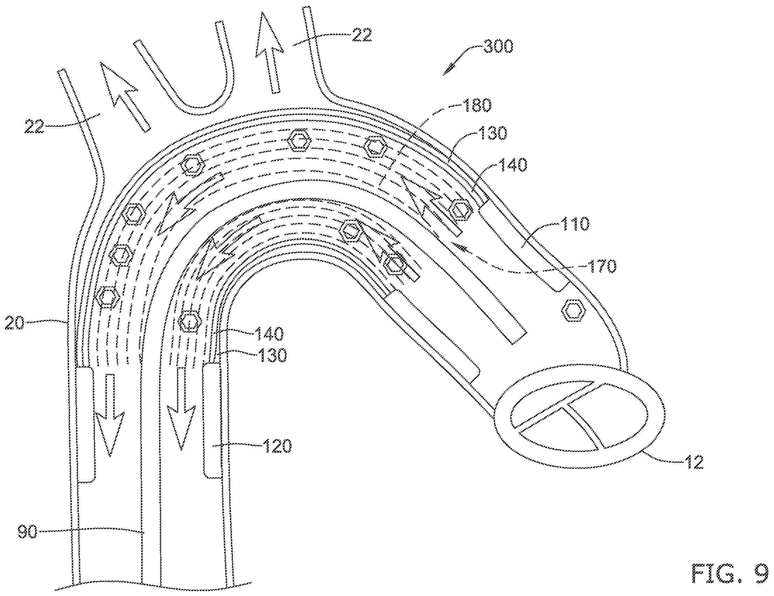

As in the example(s) described above, the second filter element 180 may include an access port 170 configured to facilitate passage of an interventional catheter 90 through the access port 170. For example, the porous media may include an aperture formed and/or cut therethrough (e.g., a slit, a hole, etc.) to act as the access port 170. The aperture in the porous media may be self-biased toward a closed configuration, wherein fluid/blood and/or thrombi and/or embolic material contained therein is unable to freely pass through the aperture. In some embodiments, advancement and/or translation of the interventional catheter 90 upstream through the aortic arch embolic protection device 100 and/or the access port 170 may urge and/or actuate the access port 170 into an open configuration. The access port 170 may be configured to compress against the exterior surface of the interventional catheter 90 when the interventional catheter 90 is advanced and/or translated through the access port 170, as shown in FIG. 9 for example. In some embodiments, when the aortic arch embolic protection device 100 is disposed within the arch of the aorta 20 or at another treatment location, with fluid/blood flowing therethrough, the access port 170 may only attain the open configuration when the interventional catheter 90 or another similar device is disposed within and/or through the access port 170, thereby urging and/or forcing the access port 170 into the open configuration.

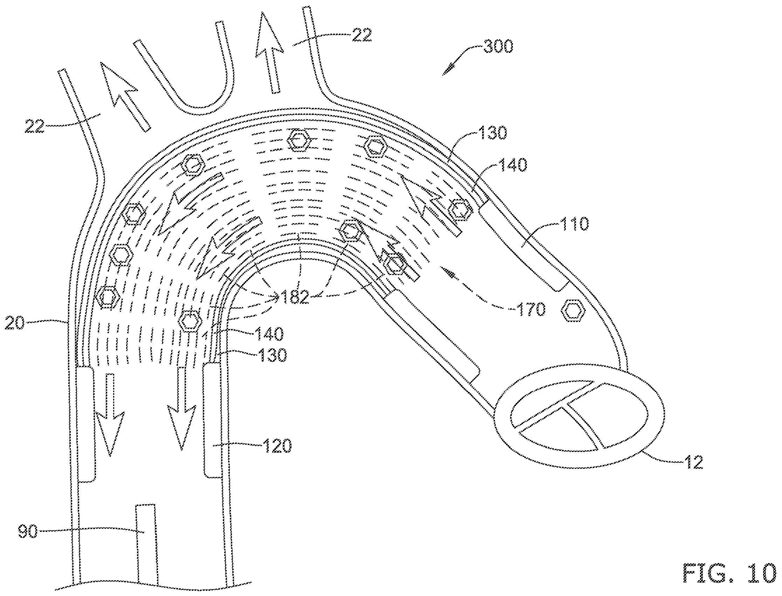

In some embodiments, the second filter element 180 may comprise a series of porous media sections 182 distributed along a length of the lumen defined by the first filter element 140. FIG. 10 illustrates an exemplary configuration of the series of porous media sections 182 in the aortic arch embolic protection device 100. Other configurations and/or arrangements are also contemplated. In some embodiments, each porous media section 182 of the series of porous media sections 182 may be axially spaced apart from an adjacent porous media section 182 along the length of the lumen defined by the first filter element 140. In some embodiments, the series of porous media sections 182 may include two porous media sections, three porous media sections, four porous media sections, five porous media sections, six porous media sections, seven porous media sections, or more porous media sections. In some embodiments, some of the series of porous media sections 182 may be immediately adjacent to each other and/or in direct contact with each other while other adjacent porous media sections 182 are axially spaced apart from each other.

The materials that can be used for the various components of the interventional catheter 90, the aortic arch embolic protection device 100/200/300, the first anchoring ring 110, the second anchoring ring 120, the tubular scaffold 130, the first filter element 140, the second filter elements 150/180, the porous media, the plurality of tethers 160, the elastic band, etc. (and/or other systems or components disclosed herein) and the various elements thereof disclosed herein may include those commonly associated with medical devices. For simplicity purposes, the following discussion makes reference to the interventional catheter 90, the aortic arch embolic protection device 100/200/300, the first anchoring ring 110, the second anchoring ring 120, the tubular scaffold 130, the first filter element 140, the second filter elements 150/180, the porous media, the plurality of tethers 160, the elastic band, etc. However, this is not intended to limit the devices and methods described herein, as the discussion may be applied to other elements, members, components, or devices disclosed herein, such as, but not limited to, the interventional catheter 90, the aortic arch embolic protection device 100/200/300, the first anchoring ring 110, the second anchoring ring 120, the tubular scaffold 130, the first filter element 140, the second filter elements 150/180, the porous media, the plurality of tethers 160, the elastic band, etc. and/or elements or components thereof.

In some embodiments, the interventional catheter 90, the aortic arch embolic protection device 100/200/300, the first anchoring ring 110, the second anchoring ring 120, the tubular scaffold 130, the first filter element 140, the second filter elements 150/180, the porous media, the plurality of tethers 160, the elastic band, etc., and/or components thereof, may be made from a metal, metal alloy, polymer (some examples of which are disclosed herein), a metal-polymer composite, ceramics, combinations thereof, and the like, or other suitable material. Some examples of suitable metals and metal alloys include stainless steel, such as 444V, 444L, and 314LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R44035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R44003 such as ELGILOY®, PHYNOX®, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear than the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Other suitable materials may include ULTANIUM™ (available from Neo-Metrics) and GUM METAL™ (available from Toyota). In some other embodiments, a superelastic alloy, for example a superelastic nitinol can be used to achieve desired properties.

In at least some embodiments, portions or all of the interventional catheter 90, the aortic arch embolic protection device 100/200/300, the first anchoring ring 110, the second anchoring ring 120, the tubular scaffold 130, the first filter element 140, the second filter elements 150/180, the porous media, the plurality of tethers 160, the elastic band, etc., and/or components thereof, may also be doped with, made of, or otherwise include a radiopaque material. Radiopaque materials are understood to be materials capable of producing a relatively bright image on a fluoroscopy screen or another imaging technique during a medical procedure. This relatively bright image aids a user in determining the location of the interventional catheter 90, the aortic arch embolic protection device 100/200/300, the first anchoring ring 110, the second anchoring ring 120, the tubular scaffold 130, the first filter element 140, the second filter elements 150/180, the porous media, the plurality of tethers 160, the elastic band, etc. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the interventional catheter 90, the aortic arch embolic protection device 100/200/300, the first anchoring ring 110, the second anchoring ring 120, the tubular scaffold 130, the first filter element 140, the second filter elements 150/180, the porous media, the plurality of tethers 160, the elastic band, etc. to achieve the same result.

In some embodiments, a degree of Magnetic Resonance Imaging (MRI) compatibility is imparted into the interventional catheter 90, the aortic arch embolic protection device 100/200/300, the first anchoring ring 110, the second anchoring ring 120, the tubular scaffold 130, the first filter element 140, the second filter elements 150/180, the porous media, the plurality of tethers 160, the elastic band, etc. For example, the interventional catheter 90, the aortic arch embolic protection device 100/200/300, the first anchoring ring 110, the second anchoring ring 120, the tubular scaffold 130, the first filter element 140, the second filter elements 150/180, the porous media, the plurality of tethers 160, the elastic band, etc., and/or components or portions thereof, may be made of a material that does not substantially distort the image and create substantial artifacts (e.g., gaps in the image). Certain ferromagnetic materials, for example, may not be suitable because they may create artifacts in an MRI image. The interventional catheter 90, the aortic arch embolic protection device 100/200/300, the first anchoring ring 110, the second anchoring ring 120, the tubular scaffold 130, the first filter element 140, the second filter elements 150/180, the porous media, the plurality of tethers 160, the elastic band, etc., or portions thereof, may also be made from a material that the MRI machine can image. Some materials that exhibit these characteristics include, for example, tungsten, cobalt-chromium-molybdenum alloys (e.g., UNS: R44003 such as ELGILOY®, PHYNOX®, and the like), nickel-cobalt-chromium-molybdenum alloys (e.g., UNS: R44035 such as MP35-N® and the like), nitinol, and the like, and others.

In some embodiments, the interventional catheter 90, the aortic arch embolic protection device 100/200/300, the first anchoring ring 110, the second anchoring ring 120, the tubular scaffold 130, the first filter element 140, the second filter elements 150/180, the porous media, the plurality of tethers 160, the elastic band, etc., and/or portions thereof, may be made from or include a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane (for example, Polyurethane 85A), polypropylene (PP), polyvinylchloride (PVC), polyether-ester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, polyolefin, polystyrene, epoxy, polyvinylidene chloride (PVdC), poly(styrene-*b*-isobutylene-*b-*styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

It should be understood that this disclosure is, in many respects, only illustrative. Changes may be made in details, particularly in matters of shape, size, and arrangement of steps without exceeding the scope of the invention. This may include, to the extent that it is appropriate, the use of any of the features of one example embodiment being used in other embodiments. The invention's scope is, of course, defined in the language in which the appended claims are expressed.

## Claims

1. An aortic arch embolic protection device (100, 200, 300) having an upstream end and a downstream end, comprising:
an anchoring structure comprising a first anchoring ring (110) at the upstream end and a second anchoring ring (120) at the downstream end;
a first filter element (140) extending from the first anchoring ring (110) to the second anchoring ring (120) and defining a lumen through the aortic arch embolic protection device (100, 200, 300); and
a second filter element (150, 180) extending transversely across the lumen of the first filter element (140) at a plurality of axial locations along a longitudinal axis of the lumen;
wherein the second filter element (150, 180) includes an access port (170) configured to facilitate passage of an interventional catheter (90) through the access port (170).

2. The aortic arch embolic protection device (300) of claim 1, wherein the second filter element (180) comprises a porous media extending along a majority of a length of the lumen.

3. The aortic arch embolic protection device (300) of claim 2, wherein the porous media defines an effective porosity over its entire length that is less than a porosity of the porous media in any given cross-section of the porous media.

4. The aortic arch embolic protection device (300) of claim 1, wherein the second filter element (180) comprises a series of porous media sections (182) distributed along a length of the lumen, wherein each porous media section (182) of the series of porous media sections (182) is spaced apart from an adjacent porous media section (182).

## Patentansprüche

1. Aortenbogen-Embolieschutzvorrichtung (100, 200, 300) mit einem Stromaufwärtsende und einem Stromabwärtsende, die aufweist:
eine Verankerungsstruktur mit einem ersten Verankerungsring (110) am Stromaufwärtsende und einem zweiten Verankerungsring (120) am Stromabwärtsende;
ein erstes Filterelement (140), das sich vom ersten Verankerungsring (110) zum zweiten Verankerungsring (120) erstreckt und ein Lumen durch die Aortenbogen-Embolieschutzvorrichtung (100, 200, 300) hindurch festlegt; und
ein zweites Filterelement (150, 180), das sich quer über das Lumen des ersten Filterelements (140) an mehreren Axialstellen entlang einer Längsachse des Lumens erstreckt;
wobei das zweite Filterelement (150, 180) einen Zugangsport (170) aufweist, der so konfiguriert ist, dass er den Durchgang eines Interventionskatheters (90) durch den Zugangsport (170) hindurch erleichtert.

2. Aortenbogen-Embolieschutzvorrichtung (300) nach Anspruch 1, wobei das zweite Filterelement (180) ein poröses Medium aufweist, das sich entlang eines Großteils einer Länge des Lumens erstreckt.

3. Aortenbogen-Embolieschutzvorrichtung (300) nach Anspruch 2, wobei das poröse Medium eine wirksame Porosität über seine Gesamtlänge festlegt, die kleiner als eine Porosität des porösen Mediums in jedem vorgegebenen Querschnitt des porösen Mediums ist.

4. Aortenbogen-Embolieschutzvorrichtung (300) nach Anspruch 1, wobei das zweite Filterelement (180) eine Folge poröser Medienteilstücke (182) aufweist, die über eine Länge des Lumens verteilt sind, wobei jedes poröse Medienteilstück (182) der Folge poröser Medienteilstücke (182) von einem benachbarten porösen Medienteilstück (182) beabstandet ist.

## Revendications

1. Dispositif de protection embolique (100, 200, 300) de l'arc aortique ayant une extrémité en amont et une extrémité en aval, comprenant :
une structure d'ancrage comprenant un premier anneau d'ancrage (110) au niveau de l'extrémité en amont et un second anneau d'ancrage (120) au niveau de l'extrémité en aval ;
un premier élément de filtre (140) s'étendant à partir du premier anneau d'ancrage (110) jusqu'au second anneau d'ancrage (120) et définissant une lumière à travers le dispositif de protection embolique (100, 200, 300) de l'arc aortique ; et
un second élément de filtre (150, 180) s'étendant de manière transversale de part et d'autre de la lumière du premier élément de filtre (140) à une pluralité d'emplacements axiaux le long d'un axe longitudinal de la lumière ;
dans lequel le second élément de filtre (150, 180) comprend un orifice d'accès (170) configuré pour faciliter le passage d'un cathéter d'intervention (90) à travers l'orifice d'accès (170).

2. Dispositif de protection embolique (300) de l'arc aortique selon la revendication 1, dans lequel le second élément de filtre (180) comprend un support poreux s'étendant le long d'une majeure partie d'une longueur de la lumière.

3. Dispositif de protection embolique (300) de l'arc aortique selon la revendication 2, dans lequel le support poreux définit une porosité effective sur toute sa longueur qui est inférieure à une porosité du support poreux dans n'importe quelle section transversale donnée du support poreux.

4. Dispositif de protection embolique (300) de l'arc aortique selon la revendication 1, dans lequel le second élément de filtre (180) comprend une série de sections de support poreux (182) réparties le long d'une longueur de la lumière, dans lequel chaque section de support poreux (182) de la série de sections de support poreux (182) est espacée d'une section de support poreux (182) adjacente.
